# EUROPÄISCHE PATENTSCHRIFT

(11) **EP 1 524 003 B1**
(45) Veröffentlichungstag und Bekanntmachung des Hinweises auf die Patenterteilung: **04.10.2006**
(21) Anmeldenummer: 04020238.4
(22) Anmeldetag: 26.08.2004
(51) Int. Cl.: A61M 16/06

(54) **Beatmungsmaske**
Respiratory mask
Masque respiratoire

(30) Priorität: 18.10.2003 DE 10348532
(43) Veröffentlichungstag der Anmeldung: 20.04.2005
(73) Patentinhaber: Weinmann Geräte für Medizin GmbH & Co. KG, 22525 Hamburg (DE)
(72) Erfinder: Eifler, Martin, 25348 Glückstadt (DE); Schulz, Gerd, 22869 Schenefeld (DE)
(74) Vertreter: Klickow, Hans-Henning

(56) Entgegenhaltungen:
- EP-A- 1 057 494
- WO-A-20/05002656
- US-A- 5 018 519
- US-A- 5 657 752
- US-A1- 2002 170 557

## Beschreibung

Die Erfindung betrifft eine Beatmungsmaske für die Beatmung eines Patienten, die einen Maskengrundkörper sowie ein zur Anlage am Gesicht eines Patienten vorgesehenes Konturelement aufweist sowie bei der in einem Übergangsbereich zwischen dem Maskengrundkörper und dem Konturelement mindestens ein Ausströmkanal für Atemgas angeordnet ist

Eine Beatmungsmaske mit einem separaten Ausatmungselement wird beispielsweise in der DE-OS 199 03 732 beschrieben. Der Maskengrundkörper ist hierbei starr in ein Kupplungselement übergeleitet, das mit dem Ausatmungselement koppelbar ist. Das Ausatmungselement ist mit einem Schlauchadapter verbindbar, der die Atemmaske über einen Beatmungsschlauch an ein Beatmungsgerät anschließt. Eine derartige Beatmungsmaske ist insbesondere dafür geeignet, bei der sogenannten CPAP-Therapie eingesetzt zu werden.

Eine weitere Beatmungsmaske wird in der DE-OS 101 58 066 beschrieben. Ein Beatmungsschlauch wird hierbei über ein kugelsegmentartiges Gelenk mit der Beatmungsmaske verbunden. Ein Ausströmkanal für Atemgas wird gemeinsam von einem Ausatmungselement einerseits und von einem mit der Maske verbundenen Kupplungselement andererseits begrenzt.

Bekannt ist es ebenfalls, ein Ausatemsystem im Bereich einer Atemmaske derart zu realisieren, daß direkt am Maskengrundkörper mehrere Ausatemöffnungen angeordnet sind, die als Löcher oder Schlitze ausgebildet sind. Hierdurch werden jedoch relativ laute Ausatemgeräusche unmittelbar im Kopfbereich des Patienten erzeugt. Dies ist insbesondere bei Anwendungen während der Nacht nicht akzeptabel.

Aus der US-A-2002/0170557 ist es bereits bekannt, auf einen bodenlosen flaschenartigen Grundkörper eine Beatmungsmaske aufzusetzen, die benachbart zu einem die Beatmungsmaske mit dem Grundkörper verbindenden stutzenartigen Kopplungselement einen Ausströmkanal für Atemgas besitzt.

Aus der US-A-5,018,519 ist eine Atemmaske zur Zufuhr eines Anästhesiegases bekannt, an die vier Atemgasschläuche anschließbar sind. Es verlaufen jeweils ein Gaszuführungsschlauch und ein Gasableitungsschlauch parallel zueinander. Zur Richtungssteuerung des Atemgases ist die Atemmaske teilweise doppelschalig ausgebildet.

Die US-A 5,657,652 beschreibt die Anordnung eines Ausströmkanals im Bereich eines Grundkörpers einer Atemmaske bzw. alternativ im Bereich eines Anschlußelementes für den Atemgasschlauch.

Aus der nachveröffentlichten WO-A-2005/002656 ist bereits eine als Nasalmaske ausgebildete Atemmaske bekannt, die einen Grundkörper aus einem relativ festen Material und ein Konturelement aus einem relativ zum Maskengrundkörper weicheren Material aufweist. In einem seitlichen Bereich des Konturelementes ist benachbart zum Maskengrundkörper ein Ausströmkanal angeordnet, durch den verbrauchtes Atemgas in eine Umgebung abgeleitet wird.

Generell sind die bislang bekannten Beatmungsmasken und Ausatemsysteme relativ laut, da die Ausatemöffnungen relativ kompakte Geometrien aufweisen und hierdurch eine ausreichend diffuse Abströmung nicht ausreichend unterstützen.

Eine Aufgabe der vorliegenden Erfindung ist es, eine Beatmungsmaske der einleitend genannten Art derart zu konstruieren, daß eine geringe Schallemission beim Ausatmen bei geringer Teileanzahl und hohem Benutzungskomfort erreicht wird.

Durch die Lokalisierung des Ausströmkanals im Übergangsbereich zwischen den Maskengrundkörper und dem Konturelement wird eine relativ langgestreckte Formgebung des Ausströmkanals unterstützt, so daß geringe Strömungsgeschwindigkeiten des Gases und damit geringe Schallemissionen unterstützt werden. Darüber hinaus wird es durch diese Lokalisierung des Ausströmkanals ermöglicht, den Ausströmkanal relativ eng zu dimensionieren, um eine diffuse Abströmung des Atemgases zu unterstützen. Aufgrund der langgestreckten Dimensionierung des Ausströmkanals führt dessen enge Dimensionierung nicht zu einer ungünstigen Erhöhung des Strömungswiderstandes, sondern es wird eine ausreichend große Ausströmfläche bereitgestellt.

Die Anordnung des Ausströmkanals im Übergangsbereich zwischen dem Maskengrundkörper und dem Konturelement unterstützt darüber hinaus auch eine günstige werkzeugtechnische Herstellung der Teile. Ebenfalls werden eine sehr hohe Funktionalität und eine effektive Auswaschung von Kohlendioxid bereitgestellt, da der Ausströmkanal relativ dicht zu einer Nase des Patienten positioniert wird.

Eine weitere Aufgabe der Erfindung ist die Vermeidung eines Verschließens des Ausströmkanals durch Verformungen der den Ausströmkanal begrenzenden Bauelemente. Diese Aufgabe wird erfindungsgemäß dadurch gelöst daß der Ausströmkanal von mindestens einem Distanzelement unterteilt ist.

Die Bereitstellung einer belastungsfähigen Begrenzung des Ausströmkanals wird dadurch unterstützt, daß der Ausströmkanal mindestens bereichsweise von einem Distanzring begrenzt ist, der zwischen dem Maskengrundkörper und dem Konturelement angeordnet ist.

Eine einfache werkzeugtechnische Herstellung wird dadurch unterstützt, daß mindestes eines der Distanzelemente im Bereich des Distanzringes angeordnet ist.

Unter Berücksichtigung typischer Materialeigenschaften erweist es sich als vorteilhaft, daß das Distanzelement im Bereich einer dem. Maskengrundkörper zugewandten Begrenzungsfläche des Distanzringes angeordnet ist.

Gemäß einer anderen Ausführungsform ist vorgesehen, daß mindestes eines der Distanzelemente im Bereich des Maskengrundkörpers angeordnet ist.

Eine weitere Fertigungsvariante besteht darin, daß das Distanzelement im Bereich einer dem Konturelement zugewandten Begrenzungsfläche des Distanzringes angeordnet ist.

Eine Formgebung unter Berücksichtigung einer typischen Gesichtsanatomie eines Patienten erfolgt dadurch, daß der Distanzring eine im wesentlichen dreieckförmige Grundfläche mit gerundeten Eckbereichen begrenzt.

Eine günstige Strömungsführung wird dadurch unterstützt, daß im Bereich mindestens eines Seitenschenkels des Distanzringes der Ausströmkanal begrenzt ist.

Die Bereitstellung eines möglichst langgestreckten Ausströmkanals wird dadurch unterstützt, daß im Bereich jeden Seitenschenkels ein Ausströmkanal begrenzt ist.

Eine typische Materialauswahl besteht darin, daß der Distanzring aus einem härteren Material als das Konturelement ausgebildet ist.

Eine einfache Montage und Handhabung wird dadurch unterstützt, daß der Distanzring und das Konturelement einteilig ausgebildet sind.

Insbesondere trägt es zu einer einfacheren Handhabung bei, daß der Distanzring am Konturelement angespritzt ist.

Eine andere Ausführungsvariante besteht darin, daß der Distanzring und das Konturelement miteinander verklebt sind.

Darüber hinaus ist daran gedacht, daß der Distanzring und das Konturelement miteinander verschweißt sind.

Ein modularer Vorrichtungsaufbau wird dadurch unterstützt, daß der Distanzring und der Maskengrundkörper relativ zueinander lösbar fixiert sind.

Eine einfache Montage wird auch dadurch unterstützt, daß der Distanzring und der Maskengrundkörper von einer Schnappverbindung miteinander gekoppelt sind.

Eine optimale Ausnutzung der vorhandenen Bauteilegeometrie wird dadurch erreicht, daß die Fixierung im Bereich von Überleitungen der Seitenschenkel angeordnet ist.

In den Zeichnungen sind Ausführungsbeispiele der Erfindung schematisch dargestellt. Es zeigen:
- Fig. 1: Eine schematische Darstellung einer Beatmungseinrichtung mit Beatmungsmaske,
- Fig. 2: eine perspektivische Darstellung eines Maskengrundkörpers der Beatmungsmaske,
- Fig. 3: eine perspektivische Darstellung des Maskengrundkörpers gemäß Figur 2 gemeinsam mit einem Distanzring sowie einem Konturelement,
- Fig. 4: eine Draufsicht gemäß Blickrichtung IV in Figur 3 und
- Fig. 5: eine schematische Darstellung des Distanzringes.

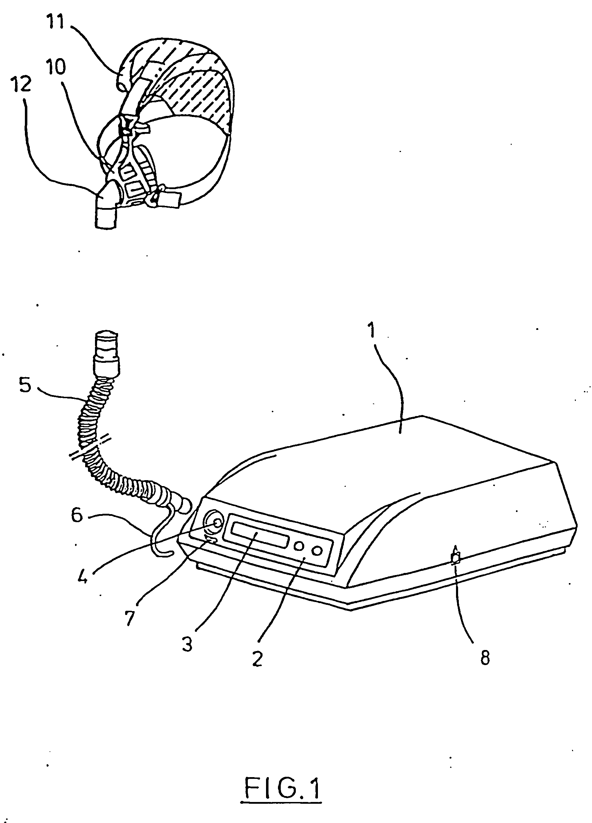

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

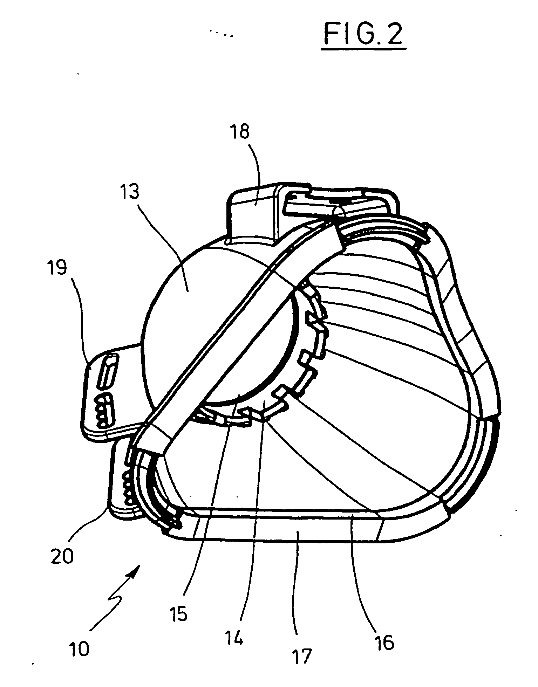

Fig. 2 zeigt einen Maskengrundkörper (13) der Beatmungsmaske (10) in einer perspektivischen Darstellung. Der Maskengrundkörper (13) weist ein Halterungselement (14) für das Kupplungselement (12) auf. Bei der in Figur 2 dargestellten Ausführungsform ist das Haltungselement (14) zur Aufnahme eines kugelgelenkartigen Teiles des Kupplungselementes (12) ausgebildet. Das Haltungselement (14) begrenzt eine Zugangsöffnung (15) des Maskengrundkörpers (13)

Der Zugangsöffnung (15) abgewandt angeordnet ist eine Durchtrittsöffnung (16), die von einem Rand (17) begrenzt ist. Der Rand (17) verläuft mit einer im wesentlichen dreieckförmigen Kontur, bei der Seitenbereiche jeweils gerundet ineinander übergeleitet sind. Figur 2 veranschaulicht ebenfalls ein Halterungselement (18) für die in Figur 1 dargestellte Kopfhaube (11) oder für vergleichbare riemenartige Halterungselemente und Halterungselemente (19, 20) für eine nicht dargestellte Stirnstütze.

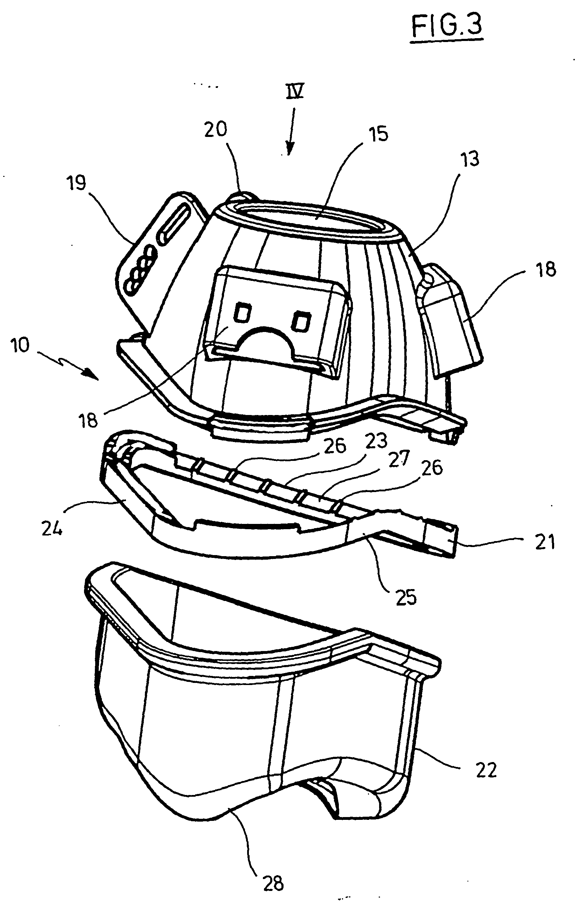

Fig. 3 zeigt in einer perspektivischen Darstellung den Maskengrundkörper (13) gemäß Figur 2 in einer gedrehten Positionierung und gemeinsam mit einem Distanzring (21) sowie einem Konturelement (22). Der Distanzring (21) weist eine an die Gestaltung des Randes (17) angepaßte Formgebung auf und spannt somit ebenfalls eine im wesentlichen dreieckförmige Grundkontur mit gerundeten Eckbereichen auf. Im Bereich von Seitenschenkeln (23, 24, 25) ist der Distanzring (21) im Bereich seiner dem Maskengrundkörper (13) zugewandten Begrenzung mit Distanzelementen (26) versehen, die im wesentlichen stegartig ausgebildet sind. Nach einem Zusammenfügen des Distanzringes (21) und des Maskengrundkörpers (13) sind zwischen den Distanzelementen (26) Bereiche eines Ausströmkanals (27) ausgebildet. Die Distanzelemente (26) erstrecken sich im wesentlichen quer zu Längsverläufen der Seitenschenkel (23, 24, 25).

Der Maskengrundkörper (13) und der Distanzring (21) werden typischerweise aus einem harten bzw. mittelharten Kunststoff ausgebildet. Das Konturelement wird aus einem relativ weichen Kunststoff ausgebildet, um eine Anlage am Gesicht des Patienten zu unterstützen. Die Flexibilität des Konturelementes (22) wird insbesondere durch Dichtlippen (28) unterstützt, die das Konturelement (22) im Bereich seiner dem Maskengrundkörper (13) abgewandten Ausdehnung aufweist.

Alternativ zur Anordnung der Distanzelemente (26) im Bereich des Distanzringes (21) können diese auch im Bereich des Randes (17) des Maskengrundkörpers (13) angeformt sein. Ebenfalls ist es denkbar, sowohl den Distanzring (21) als auch den Maskengrundkörper (13) im Bereich ihrer einander jeweils zugewandten Ausdehnungen mit Distanzelementen (26) zu versehen.

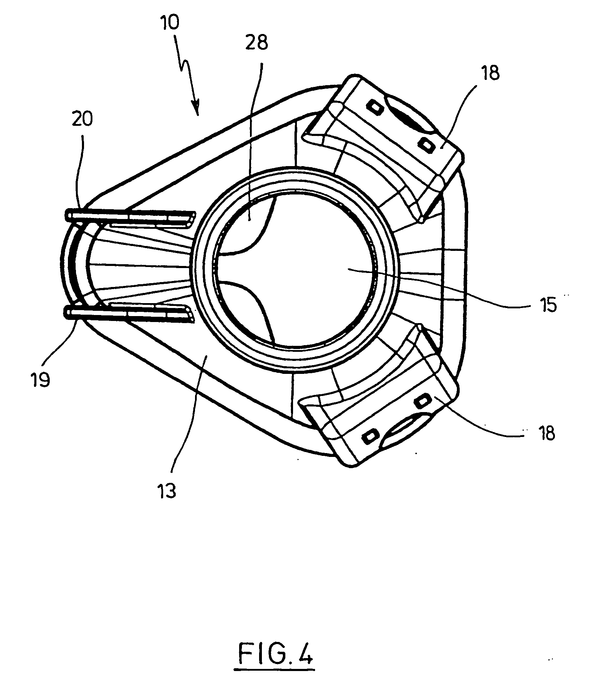

Fig. 4 veranschaulicht nochmals die Gestaltung des Maskengrundkörpers (13). Durch die Zugangsöffnung (15) hindurch sind darüber hinaus Teile der Dichtlippen (28) des Konturelementes (22) zu erkennen.

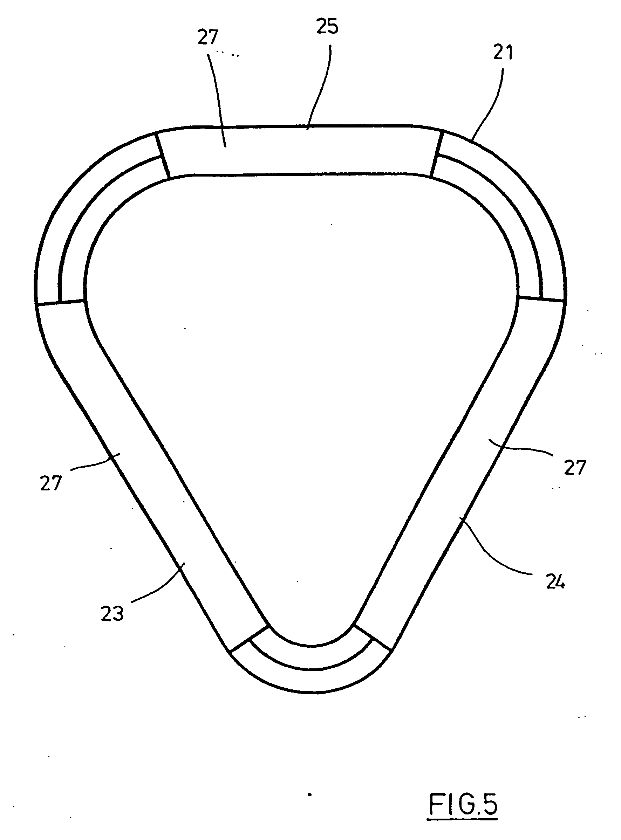

Fig. 5 zeigt eine Draufsicht auf den Distanzring (21). Im Bereich der Seitenschenkel (23, 24, 25) sind jeweils Flächen zur Begrenzung der Ausströmkanäle (27) eingezeichnet. Die Distanzelemente (26) sind in Figur 5 nicht dargestellt.

## Patentansprüche

1. Beatmungsmaske für die Beatmung eines Patienten, die einen Maskengrundkörper (13) sowie ein zur Anlage am Gesicht eines Patienten vorgesehenes Konturelement (22) aufweist sowie bei der in einem Übergangsbereich zwischen dem Maskengrundkörper (13) und dem Konturelement (22) mindestens ein Ausströmkanal (27) für Atemgas angeordnet ist, **dadurch gekennzeichnet, daß** der Ausströmkanal (27) von mindestens einem Distanzelement (26) unterteilt ist.

2. Beatmungsmaske nach Anspruch 1, **dadurch gekennzeichnet, daß** der Ausströmkanal (27) mindestens bereichsweise von einem Distanzring (21) begrenzt ist, der zwischen dem Maskengrundkörper (13) und dem Konturelement (22) angeordnet ist.

3. Beatmungsmaske nach Anspruch 1 oder 2, **dadurch gekennzeichnet, daß** mindestes eines der Distanzelemente (26) im Bereich des Distanzringes (21) angeordnet ist.

4. Beatmungsmaske nach Anspruch 3, **dadurch gekennzeichnet, daß** das Distanzelement (26) im Bereich einer dem Maskengrundkörper (13) zugewandten Begrenzungsfläche des Distanzringes (21) angeordnet ist.

5. Beatmungsmaske nach einem der Ansprüche 1 bis 4, **dadurch gekennzeichnet, daß** mindestes eines der Distanzelemente (26) im Bereich des Maskengrundkörpers (13) angeordnet ist.

6. Beatmungsmaske nach Anspruch 5, **dadurch gekennzeichnet, daß** das Distanzelement (26) im Bereich einer dem Konturelement (22) zugewandten Begrenzungsfläche des Distanzringes (21) angeordnet ist.

7. Beatmungsmaske nach einem der Ansprüche 1 bis 6, **dadurch gekennzeichnet, daß** der Distanzring (21) eine im wesentlichen dreieckförmige Grundfläche mit gerundeten Eckbereichen begrenzt.

8. Beatmungsmaske nach einem der Ansprüche 1 bis 7, **dadurch gekennzeichnet, daß** im Bereich mindestens eines Seitenschenkels (23, 24, 25) des Distanzringes (21) der Ausströmkanal (27) begrenzt ist.

9. Beatmungsmaske nach Anspruch 8, **dadurch gekennzeichnet, daß** im Bereich jeden Seitenschenkels (23, 24, 25) ein Ausströmkanal (27) begrenzt ist.

10. Beatmungsmaske nach einem der Ansprüche 1 bis 9, **dadurch gekennzeichnet, daß** der Distanzring (21) aus einem härteren Material als das Konturelement (22) ausgebildet ist.

11. Beatmungsmaske nach einem der Ansprüche 1 bis 10, **dadurch gekennzeichnet, daß** der Distanzring (21) und das Konturelement (22) einteilig ausgebildet sind.

12. Beatmungsmaske nach Anspruch 11, **dadurch gekennzeichnet, daß** der Distanzring (21) am Konturelement (22) angespritzt ist.

13. Beatmungsmaske nach Anspruch 11, **dadurch gekennzeichnet, daß** der Distanzring (21) und das Konturelement (22) miteinander verklebt sind.

14. Beatmungsmaske nach Anspruch 11, **dadurch gekennzeichnet, daß** der Distanzring (21) und das Konturelement (22) miteinander verschweißt sind.

15. Beatmungsmaske nach einem der Ansprüche 1 bis 10, **dadurch gekennzeichnet, daß** der Distanzring (21) und der Maskengrundkörper (13) relativ zueinander lösbar fixiert sind.

16. Beatmungsmaske nach Anspruch 15, **dadurch gekennzeichnet, daß** der Distanzring (21) und der Maskengrundkörper (13) von einer Schnappverbindung miteinander gekoppelt sind.

17. Beatmungsmaske nach Anspruch 15, **dadurch gekennzeichnet, daß** die Fixierung im Bereich von Überleitungen der Seitenschenkel (23, 24, 25) angeordnet ist.

## Claims

1. Respiratory mask to assist the breathing of a patient, comprising a mask base body (13) and a contour element (22) for fitting on the face of a patient, and at least one outlet passage (27) for respiratory gas is provided in a transition region between the mask base body (13) and the contour element (22), **characterised in that** the outlet passage (27) is divided by at least one spacer element (26).

2. Respiratory mask as claimed in claim 1, **characterised in that** at least certain regions of the outlet passage (27) are bounded by a spacer ring (21) disposed between the mask base body (13) and the contour element (22).

3. Respiratory mask as claimed in claim 1 or 2, **characterised in that** at least one of the spacer elements (26) is disposed in the region of the spacer ring (21).

4. Respiratory mask as claimed in claim 3, **characterised in that** the spacer element (26) is disposed in the region of a boundary surface of the spacer ring (21) facing the mask base body (13).

5. Respiratory mask as claimed in one of claims 1 to 4, **characterised in that** at least one of the spacer elements (26) is disposed in the region of the mask base body (13).

6. Respiratory mask as claimed in claim 5, **characterised in that** the spacer element (26) is disposed in the region of a boundary surface of the spacer ring (21) facing the contour element (22).

7. Respiratory mask as claimed in one of claims 1 to 6, **characterised in that** the spacer ring (21) bounds an essentially triangular-shaped base surface with rounded corner regions.

8. Respiratory mask as claimed in one of claims 1 to 7, **characterised in that** the outlet passage (27) is bounded in the region of at least one side leg (23, 24, 25) of the spacer ring (21).

9. Respiratory mask as claimed in claim 8, **characterised in that** an outlet passage (27) is bounded in the region of each side leg (23, 24, 25).

10. Respiratory mask as claimed in one of claims 1 to 9, **characterised in that** the spacer ring (21) is made from a harder material than the contour element (22).

11. Respiratory mask as claimed in one of claims 1 to 10, **characterised in that** the spacer ring (21) and the contour element (22) are made as an integral part.

12. Respiratory mask as claimed in claim 11, **characterised in that** the spacer ring (21) is injected onto the contour element (22).

13. Respiratory mask as claimed in claim 11, **characterised in that** the spacer ring (21) and the contour element (22) are adhered to one another.

14. Respiratory mask as claimed in claim 11, **characterised in that** the spacer ring (21) and the contour element (22) are welded to one another.

15. Respiratory mask as claimed in one of claims 1 to 10, **characterised in that** the spacer ring (21) and the mask base body (13) are detachably fixed relative to one another.

16. Respiratory mask as claimed in claim 15, **characterised in that** the spacer ring (21) and the mask base body (13) are coupled with one another by means of a snap-fit connection.

17. Respiratory mask as claimed in claim 15, **characterised in that** the fixture is disposed in the region of the transitions between the side legs (23, 24, 25).

## Revendications

1. Masque de respiration artificielle pour la respiration d'un patient, qui présente un corps de base de masque (13) ainsi qu'un élément de contour (22) prévu pour l'ajustement au visage du patient, ainsi que dans lequel, dans la zone de transition entre le corps de base de masque (13) et l'élément de contour (22), au moins un canal d'évacuation (27) pour le gaz de respiration est agencé, **caractérisé en ce que** le canal d'évacuation (27) est divisé d'un élément écarteur (26).

2. Masque de respiration artificielle selon la revendication 1, **caractérisé en ce que** le canal d'évacuation (27) est limité au moins partiellement, par un anneau écarteur (21), qui est agencé entre le corps de base de masque (13) et l'élément de contour (22).

3. Masque de respiration artificielle selon la revendication 1 ou 2, **caractérisé en ce qu'**au moins un des éléments écarteurs (26) est agencé au niveau de l'anneau écarteur (21).

4. Masque de respiration artificielle selon la revendication 3, **caractérisé en ce que** l'élément écarteur (26) est agencé au niveau d'une surface limite de l'anneau écarteur (23), orientée vers le corps du masque (13).

5. Masque de respiration artificielle selon l'une des revendications 1 à 4, **caractérisé en ce qu'**au moins un des éléments écarteurs (26) est agencé au niveau du corps de base de masque (13).

6. Masque de respiration artificielle selon la revendication 5, **caractérisé en ce que** l'élément écarteur (26) est agencé au niveau d'une surface limite de l'anneau écarteur (23), orientée vers l'élément de contour (22).

7. Masque de respiration artificielle selon l'une des revendications 1 à 6, **caractérisé en ce que** l'anneau écarteur (21) limite une surface de base essentiellement triangulaire, avec coins arrondis.

8. Masque de respiration artificielle selon l'une des revendications 1 à 7, **caractérisé en ce que** le canal d'évacuation (27) est limité au niveau d'au moins une traverse latérale (23, 24, 25) de l'anneau écarteur (21).

9. Masque de respiration artificielle selon la revendication 5, **caractérisé en ce qu'**un canal d'évacuation (27) est limité au niveau de chaque traverse latérale (23, 24, 25).

10. Masque de respiration artificielle selon l'une des revendications 1 à 9, **caractérisé en ce que** l'anneau écarteur (21) est formé d'un matériau plus dur que l'élément de contour (22).

11. Masque de respiration artificielle selon l'une des revendications 1 à 10, **caractérisé en ce que** l'anneau écarteur (21) et l'élément de contour (22) sont d'une pièce.

12. Masque de respiration artificielle selon la revendication 11, **caractérisé en ce que** l'anneau écarteur (21) est coulé par injection sur l'élément de contour (22).

13. Masque de respiration artificielle selon la revendication 11, **caractérisé en ce que** l'anneau écarteur (21) et l'élément de contour (22) sont collés l'un à l'autre.

14. Masque de respiration artificielle selon la revendication 11, **caractérisé en ce que** l'anneau écarteur (21) et l'élément de contour (22) sont soudés l'un à l'autre.

15. Masque de respiration artificielle selon l'une des revendications 1 à 10, **caractérisé en ce que** l'anneau écarteur (21) et le corps de base de masque (13) sont fixés de manière amovible l'un par rapport à l'autre.

16. Masque de respiration artificielle selon la revendication 15, **caractérisé en ce que** l'anneau écarteur (21) et le corps de base de masque (13) sont couplés l'un à l'autre par un assemblage à enclenchement.

17. Masque de respiration artificielle selon la revendication 15, **caractérisé en ce que** la fixation est agencée au niveau des transitions des traverses latérales (23, 24, 25).
